(19) Europäisches Patentamt / European Patent Office / Office européen des brevets

(11) **EP 2 396 079 B1**

(12) **EUROPEAN PATENT SPECIFICATION**

(45) Date of publication and mention
of the grant of the patent:
**28.08.2013 Bulletin 2013/35**

(51) Int Cl.:
*A61N 5/04* (2006.01)    *A61B 18/14* (2006.01)
*A61B 18/12* (2006.01)    *A61B 18/18* (2006.01)
*A61B 5/053* (2006.01)

(21) Application number: **10703196.5**

(22) Date of filing: **08.02.2010**

(86) International application number:
**PCT/GB2010/000220**

(87) International publication number:
**WO 2010/092328 (19.08.2010 Gazette 2010/33)**

(54) **APPARATUS FOR LOCALISED INVASIVE SKIN TREATMENT USING ELECTROMAGNETIC RADIATION**

GERÄT ZUR LOKALISIERTEN INVASIVEN HAUTBEHANDLUNG MIT ELEKTROMAGNETISCHER STRAHLUNG

APPAREIL POUR TRAITEMENT CUTANÉ INVASIF LOCALISÉ À L'AIDE D'UN RAYONNEMENT ÉLECTROMAGNÉTIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.02.2009 GB 0902174**

(43) Date of publication of application:
**21.12.2011 Bulletin 2011/51**

(73) Proprietor: **Bangor University
Bangor
Gwynedd LL57 2DG (GB)**

(72) Inventor: **HANCOCK, Christopher, Paul
Bristol BS5 8QZ (GB)**

(74) Representative: **Delaney, Jennifer et al
Appleyard Lees
The Lexicon
Mount Street
Manchester M2 5NT (GB)**

(56) References cited:
**WO-A1-98/11834          WO-A1-2005/115235
WO-A1-2008/043997     WO-A1-2008/044000
WO-A2-2004/047659     WO-A2-2006/078862
WO-A2-2008/043999     WO-A2-2008/044013
WO-A2-2008/071914     US-A1- 2008 125 775**

**Description**

TECHNICAL FIELD

This application relates to apparatus for and methods of measuring and/or treating skin tissue and sub-structures within the skin.

BACKGROUND OF THE INVENTION

There are various types of known skin treatment systems, e.g. including laser treatment systems, low energy plasma treatment systems, mechanical dermabrasion, low frequency RF electrosurgical treatment systems operating at frequencies of around 500 kHz, infrared light based systems, or treatment systems using creams introduced onto the surface of the skin to penetrate through the skin, or orally introduced drugs.

There are a number of laser based skin treatment systems currently on the market; these tend to concentrate on applications in the field of cosmetic treatments where skin rejuvenation and wrinkle removal are of primary interest. Example systems include Er-YAG lasers, $CO_2$ lasers, Nd-YAG lasers, semiconductor lasers (for example, GaAs laser diodes), and Q-switched Ruby lasers.

As a first order representative qualitative model, the skin may be considered to be a plant that grows from the bottom upwards. From this model it will be understood that if interference is caused to the growth process then problems will arise, for example, if it is bombarded with ultra violet radiation from the sun, or hazardous chemicals are introduced into or onto it then, like a plant, it will become diseased and will be damaged or will eventually die if a course of treatment is not provided.

However, there are some clinical conditions which are not suited to the above treatment techniques and which are currently treated with medication that offers only a very primitive and short term solution. For example, alopecia areata is an autoimmune disease where the body's immune system mistakenly attacks hair follicles, which are the part of skin tissue from which hairs grow. If this condition arises, the hair normally falls out in small round patches. There are currently no drugs available that have been approved to treat alopecia areata and there is currently no cure for the disease.

WO 2008/071914 discloses a portable electrosurgical system for ablating biological tissue using microwave radiation.

WO 2004/047659 discloses apparatus for ablating biological tissue using microwave radiation in which an impedance adjuster is used to minimise the amount of microwave radiation reflected from the probe.

US 2008/0125775 discloses tissue treatment apparatus comprising an array of bipolar electrodes and a mechanism for selectively advancing the electrode array into a target tissue site.

WO 2006/078862 discloses tissue resection apparatus comprising an insertable electrode array for delivering energy into biological tissue.

WO 2008/043997 discloses a surgical antenna for delivering microwave radiation, the antenna having a structure that facilitates impedance matching with target tissue.

WO 2008/043999 discloses apparatus for measuring or ablating tissue with microwave radiation, the apparatus having a channel for delivering microwave radiation in a measurement mode that bypasses the amplifier used to generate microwave radiation in an ablation mode.

WO 2008/044000 discloses a surgical instrument for resecting biological tissue using microwave radiation.

WO 2005/115235 discloses apparatus for measuring and ablating biological tissue with microwave radiation.

WO 2008/044013 discloses a needle-type biopsy instrument for measuring or ablating tissue with microwave radiation.

SUMMARY OF THE INVENTION

According to the invention, there is provided skin treatment apparatus as set out in claim 1. The invention thus provides a minimally invasive treatment system for direct, localised delivery of millimetre or sub-millimetre wavelength radiation into skin tissue. At the radiation frequencies contemplated for use with the invention, the depth of penetration of energy by radiation is very small. The depth of penetration decreases with increasing frequency and relative permittivity of the tissue structure of interest. In combination with the direct delivery mechanism of the invention this may permit accurate treatment of target structures within the skin itself.

With the above apparatus, the physical location of the emitted radiation field may be confined accurately through the position of the radiating portion. The frequency used for the radiation is high, which may permit the emitted radiation to be constrained to treat structures in close proximity to the radiating portion. This permits better targeting of the power than in surface-based (i.e. non-invasive) arrangements.

Furthermore, the use of frequencies of 10 GHz or more means that the radiating portion may work efficiently with a length of 1 mm or less. Consequently, the entire invasive structure may be less than 2 mm in length, e.g. 1-2 mm, which can cause minimal patient discomfort (e.g. a slight pricking or tingly sensation). For example, the quarter wavelength of a monopole antenna loaded with wet skin at an operating frequency of 100 GHz is 0.28 mm. In this particular arrangement, only the monopole is inserted into the skin. In terms of the diameter of the radiating section, the monopole may be less than 0.5 mm in outer diameter and may take the form of a acupuncture needle.

The radiating portion may emit radiation into localised structures within the subcutaneous layer or dermis. For example, the apparatus may be used to deliver energy to and confine that energy within a sweat gland in the subcutaneous layer or a sebaceous gland in the dermis.

The apparatus may comprise a plurality of monopole antennas, e.g. arranged in an array, each monopole antenna having an invasive structure, wherein a plurality of invasive structures may be simultaneously insertable into the skin tissue. The invasive structures may be so small that it is possible to have more than one inserted inside a sub-structure of skin tissue, e.g. a sweat gland, at the same time.

The radiating portion of the antennas may be configured to be impedance matched with the skin tissue to be treated. The microwave energy may thus be efficiently transferred into the skin structure. The input or the proximal end of the antennas and the output of the signal generator may also be well matched in terms of impedance to ensure that the power delivered from the signal generator is efficiently transferred into the antenna, which will ensure that this energy is absorbed by the biological tissue that is in contact with the radiating section of the antenna.

Alternatively, this arrangement permits localised treatment of sub-structures within the skin tissue over an area of skin, e.g. for a condition such as acne.

The energy delivered by the field of radiation may be controllable. The apparatus may include a delivered power detector connected between the signal generator and antenna and arranged to detect the amount of power (and hence energy) delivered from the antenna. The apparatus may include a controller (e.g. microprocessor or digital signal processor) connected to receive information from the delivered power detector. The controller may be connected to the signal generator to control the power level of the outputted electromagnetic signal based on information from the delivered power detector. The signal generator may include a variable attenuator, operable by the controller, for controlling the output power level.

The delivered power detector may include a forward directional coupler and a reverse directional coupler connected between the signal generator and the or each antenna.

The outputs from the directional couplers may be used to calculate the magnitude of delivered power. This can be accurately controlled using the variable attenuator. The radiation disclosed herein can instantly elevate skin temperature in an extremely localised manner. The variable attenuator and directional couplers permit precise control of this tissue heating.

A circulator may be connected between the couplers and signal generator to isolate the signal generator from signals that are reflected from the antenna(s).

The signal generator may include a stable, low power, source oscillator, e.g. a voltage-controlled oscillator (VCO) or a dielectric resonator oscillator (DRO) and one or more power amplifiers. Advances in the field of microwave and millimetre wave monolithic integrated circuits (MMICs) mean that small scale devices, e.g. based on indium phosphide (InP) High Electron Mobility Transistors (HEMTs), are now available to generate high frequency signals at high power levels. Using such devices, the level of power delivered by the antenna may be between 10 mW and 2 W.

The power amplifiers may include such devices. Other similar technologies are also suitable, and are discussed below. These small scale devices make realistic embodiments of the invention possible because the power generation (amplification) can be located in close proximity to the radiating structures which can reduce or make manageable power losses.

In embodiments with a plurality of antennas, a single signal generator e.g. source oscillator and amplifier(s) may provide power to a plurality of antennas, e.g. using a suitable power splitting arrangement, i.e. waveguide splitter, microstrip splitter, 3dB coupler, Lange coupler. In other embodiments each antenna may have its own signal generator. The power delivered for each antenna may therefore be independently controllable. A single source oscillator may provide a base signal for a plurality of signal generators.

In the invention, the apparatus is arranged to measure properties of skin tissue, e.g. to determine the type of tissue (or sub-structure of the skin) that is present at the radiating portion before treatment (e.g. delivery of power of 10 mW or more) commences. The small depth of penetration of the microwave energy into the tissue offers advantage both in terms of treating small tissue structures and in identifying characteristics of fine tissue structures. The controller may be arranged to detect the magnitude and phase of the signal reflected from the antenna. The detected magnitude and phase information may be used to calculate a complex impedance value for the tissue at the radiating portion, which complex impedance value is indicative of the tissue type. The magnitude and phase may be detected using a heterodyne detector connected to the reverse directional coupler. This arrangement may also be used to diagnose a number of diseases or clinical conditions associated with various anatomical structures associated with the skin or other tissue types where small needle antenna structures may be introduced.

A reference signal for the heterodyne detector may be derived from the same source oscillator as the forward (output) radiation from the signal generator. There may be a multi-down conversion arrangement to reduce the frequency of reflected radiation to a level at which magnitude and phase information can be measured.

Preferably, the energy delivered to the tissue when the apparatus is arranged to measure tissue properties is much less (e.g. two or more orders of magnitude less) than when the apparatus is arranged to treat the tissue. The apparatus may have a treatment made and a measurement mode, wherein the power delivered is greater than 10 mW and less than 1 mW respectively. The amount of power delivered during the measurement mode is preferably less than that required to cause permanent tissue damage. The apparatus may have two channels for the output radiation: a treatment channel which includes the power amplifier and a measurement channel which bypasses the power amplifier.

The apparatus may include a switch (e.g. operable by a surgeon) to switch the output radiation between channels. With this arrangement, the device may be used to determine that the radiating portion of the invasive structure (e.g. the antenna) is in a desired tissue type before treatment begins. The localisation of the emitted field made possible by using high microwave frequency radiation is also beneficial in the measurement mode because the reflected signal is dominated by the tissue close to the radiating portions; reflection and scattering from neighbouring tissue may be negligible.

Where there is an array of insertable monopole antennas, each antenna may have an independently controllable dual channel arrangement similar to the one described above. Thus, each antenna may operate in either the treatment mode or measurement mode independently of its neighbours. This is useful for targeting specific structures (e.g. glands) within the tissue without necessarily having to direct or locate an individual antenna accurately into position. The antennas which are determined to be in the tissue type to be treated can be switched to treatment mode while the antennas determined not be in the tissue type to be treated may be switched off or left in measurement mode. The antennas in the array may then be selectively activated in accordance with measured information.

In the invention, the antenna is mechanically insertable and retractable from the skin tissue. Where there is an array of antennas, each antenna may be independently insertable and retractable. In this case, antennas that are determined not to be in the tissue type to be detected can be withdrawn from the tissue altogether. This can reduce patient discomfort.

The invasive or minimally invasive structure of each monopole antenna may include a needle or pin. The radiating portion may be the tip of the needle. The needle antenna structure may be a co-axial line of fixed impedance, for example 25 $\Omega$, 50 $\Omega$, or 75 $\Omega$. To realise physically co-axial structures with a very small outer diameter, for example 0.1 mm to 0.5 mm, it may be necessary to make use nanotechnology in order to manufacture such small size needle structures. For example, deep reactive ion etching may be used to fabricate arrays of needle antennas with a needle length of between 0.1 mm and 1 mm. Micromachining techniques may also be considered for the fabrication process.

The measured magnitude and phase information may be used to calculate a complex impedance value of the tissue at the radiating portion, which complex impedance value may be indicative of the tissue (or skin sub-structure) type. The phase and magnitude information may be manipulated in other ways to enable information such as complex permittivity, dielectric constant, or tissue conductivity data to be extracted. A three-port circulator may be connected at a junction between the signal generator, monopole antenna and detector. The output measurement signal may be input to a first port of the circulator and output at a second port which is connected to the monopole antenna. The reflected signal may thus be input to the second port and diverted to or routed to or output at a third port which is connected to the detector. With this configuration the forward (measurement) signal is isolated from the detector and the reflected signal may be provided directly to the detector (i.e. without the use of couplers) so that a low power level (e.g. 1 mW or less) can be used for the measurement signal, i.e. the amplitude of the reflected measurement signal is not reduced by a coupling factor of a directional coupler in the line-up. A low power level reduces or minimises the risk of damage to the skin tissue during measurement.

The limited depth of penetration of the frequencies described herein means the measurements are confined to skin tissue, i.e. the reflected signal is dominated by the properties of the tissue at the radiating portion. Given the size of structures of interest within the skin, it may be advantageous to use frequencies of 45 GHz or more, preferably 100 GHz or more. Enhanced measurement accuracy and sensitivity may be obtained using frequencies of 1 THz or more.

The measurement aspect of the invention may also be useful for analysing the content of solid or liquid tissue samples to monitor levels or concentrations of constituents of the samples. For example, the invention may be used to analyse urine or blood samples.

Where a plurality of monopole antennas (e.g. needle antennas) is used, each needle may be mounted on a pad of biocompatible material. The invasive or minimally invasive structures (e.g. needles) may themselves be made from a biocompatible material, or may be coated with a biocompatible material in order to ensure that the structure causes no contamination when introduced inside the body. For example, the needles may be coated with a thin layer of Parylene C.

BRIEF DESCRIPTION OF THE DRAWINGS

Examples of the invention are discussed in detail below with reference to the accompanying drawings, in which:

Fig. 1 is a schematic cross-sectional view through skin tissue having two needle antennas inserted therein;
Fig. 2 is a block diagram showing the components of a single channel skin treatment apparatus that is an embodiment of the invention;
Fig. 3 is a block diagram showing the components of a dual channel skin treatment apparatus that is an embodiment of the invention;
Fig. 4 is a block diagram showing details of a signal generator for the skin treatment apparatus shown in Fig. 2;
Fig. 5 is a block diagram showing details of a signal generator for the skin treatment apparatus shown in Fig. 3;
Fig. 6 is a side view of a monopole antenna that can be used with an embodiment of the invention;
Fig. 7 is a perspective side view of an array of monopole antennas that can be used with an embodiment of the invention;
Fig. 8 is a block diagram of a multi-antenna apparatus according to the invention where a plurality of antennas share a common source of radiation;
Fig. 9 is a block diagram of a multi-antenna apparatus according to the invention where each antenna has an independent source of radiation; and
Fig. 10 is a side view of a handheld applicator that is an embodiment of the invention.

DETAILED DESCRIPTION; FURTHER OPTIONS AND PREFERENCES

The embodiments discussed below make use of the ability to generate high microwave (e.g. sub-millimetre and millimetre) energy up to terahertz (THz) frequencies using solid state device technology. If such microwave, millimetre wave or sub-millimetre wave energy is used to excite short monopole antenna structures (e.g. needle antennas), the complete radiating antenna structure may have a length of less than 1 mm.

In this specification references to high microwave, sub-millimetre and millimetre wavelengths is a reference to a frequency range of between 10 GHz and 5 THz (5000 GHz). A preferred range is between 30 GHz and 200 GHz. The invention may also be implemented at spot frequencies e.g. of 45 GHz, 77 GHz, 94 GHz, 96 GHz, 110 GHz, 170 GHz and 200 GHz.

The invention draws on the fact that such high frequencies produce depths of penetration of radiation that may be suitable for treating certain clinical conditions relating to the structure of the skin, where the skin is a complex organ within the human body that contains a number of intricate structures.

This invention may be used to treat skin viruses and, possibly, other viruses when operating the system at the higher end of the frequency spectrum disclosed herein. Treatment using the invention may change the DNA structure of a virus in order to deactivate the virus (i.e. may prevent its DNA structure from changing further).

The invention may be used in conjunction with a device for non-invasive skin treatment, e.g. a device that uses a single patch or an array of patches to apply energy at the skin surface.

The invention may be embodied as a very high microwave frequency, or mm-wave frequency, or a sub-mm wave frequency minimally invasive skin treatment system that uses a single needle antenna or a plurality of needle antenna structures, and a single or a plurality of semiconductor devices capable of generating enough energy at an appropriate frequency to cause desired skin effects.

The invention may be used for the treatment of benign skin tumours e.g. actinic keratosis, skin tag, cutaneous horn, seborrhoeic keratosis, or general warts. The invention may be used to treat malignant tumours of the skin. The invention may treat all structures of the skin, including skin cells, blood vessels, the nervous system and the immune system of the skin. The system may therefore be effective for treating the following conditions that relate to the skin: pyoderma gangrenosum, vitiligo, prurigo, alopecia areata, localized morphea, hypertrophic scar and keloid, etc. The invention may also be used for relief of chronic pain - postherpetic neuralgia (PHN). The frequency and the power level may be selected depending on the desired treatment. The apparatus of the instrument may therefore be used to treat or destroy a number of conditions associated with the skin. Some specific uses are explained below.

A particular clinical use of the invention may be the treatment of atopic and seborrhoeic dermatitis or acne, where over-activity of the sebaceous or sweat glands cause excessive sweating, which can lead to bacteria or fungus forming on the surface of the skin. The fungus produced is known as pityrosporum, which is a common bacterium that forms on the skin and manifests in regions where people sweat, for example, the head, under the breast, the forehead, and the armpits. Since people with sebhorrheic dermatitis produce more sweat than normal this leads to more pityrosporum fungus being produced. A single needle antenna or an array of needle antennas as discussed below may be inserted into the pores of the skin and into a sebaceous or sweat gland, where the desired treatment depth may be located between 1 mm and 2 mm from the surface of the skin (this is dependent upon the region of the body and the age of the patient), and a microwave or millimetre wave power source may be activated to deliver a controlled dose of energy into the gland to inhibit the excessive activity. Pin antenna structures may be employed in such an arrangement to launch controlled high frequency microwave energy into the pores or sweat glands. For example, pin antennas with outside diameters of less than 0.15 mm, and lengths of less than 1 mm, coupled with small depths of penetration of radiation produced by the antenna may be used.

It may be undesirable to launch energy into the hair follicle as this may cause damage to the following structures that form the hair follicle: the cuticle, Huxley's layer, Henle's layer, the external sheath, the glassy membrane and the connective layer. It may be desirable to use the measurement aspect of the invention to ensure that the needle is not located inside the hair follicle before higher energy is applied that will alter the state or cause a permanent change to the structure. The measurement aspect of the invention may permit differentiation between the hair follicle and the sebaceous and sweat glands. In one embodiment, the combined measurement or identification and selective high energy delivery features may be used permanently to remove hair from regions of the body. In other embodiments, if the measured complex impedance or other dielectric information obtainable from the magnitude and phase of the reflected signal indicates that the needle is located inside the hair follicle, an alarm condition may be flagged or activated to indicate to the surgeon that the needle should be removed. The needle could be removed manually or automatically. In the latter case, a mechanical mechanism could be activated to remove the needle and the decision to send the activation signal is based on the measured tissue information. A mechanism could be provided to prevent the system from delivering energy when a certain range of tissue impedance values are measured.

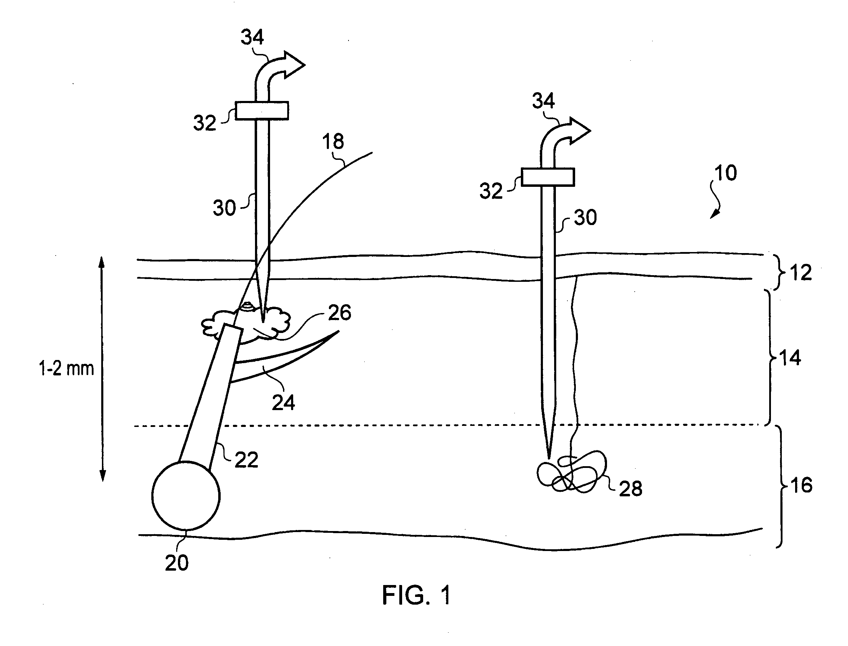

Fig. 1 shows a schematic cross-sectional view of the structure of the skin 10 and gives an illustrative view of two possible uses of the invention. The skin 10 can be considered to comprise three layers: the epidermis 12, the dermis 14, and the subcutaneous layer 16. A hair shaft 18 protrudes through a pore (not shown) in the epidermis 12 to be exposed on the outside of the skin 10. The hair shaft 18 is part of a complex structure that includes a hair matrix 20 in the subcutaneous layer 16, a hair follicle 22 which extends into the dermis 14, an arrector pili muscle 24 for erecting the hair follicle 22, and a sebaceous gland 26. A sweat gland 28 extending from the subcutaneous layer 16 to a pore in the epidermis 12 is also shown.

Fig. 1 shows two needle antenna structures 30 introduced through the surface of the skin tissue 10. One antenna is inserted into the sweat gland 28 and another antenna is inserted into the sebaceous gland 26. Such an arrangement may be useful for treating acne or seborrhoeic dermatitis, where the sebaceous glands and the sweat glands are overactive and produce an excess of sweat that leads to the formation of bacteria on the surface of the skin. The antenna structures 30 each have a microwave connector 32 at their proximal end which is arranged to transmit high frequency microwave radiation (e.g. sub-millimetre wave radiation or millimetre wave radiation) to and from the antenna structure 30 via feed structure 34. The distal end of each antenna structure comprises an invasive structure; in this embodiment the invasive structure comprises a needle point. This facilitates insertion of the antenna into the skin tissue. The invasive structure also includes a radiation portion, in this embodiment the tip of the needle, at which the energy transmitted to the antenna may be emitted into the tissue.

At the frequencies disclosed herein, e.g. 10 GHz or more, the emitted radiation field has a very small depth of penetration, so the energy introduced by the antenna can be confined locally to the sweat gland 28 and sebaceous gland 26. The localisation of the energy means that the hair structure, e.g. the hair follicle 22, may be unaffected during treatment. This is advantageous because if the hair follicle is destroyed then this will lead to swelling to the surface of the skin, which is an undesirable effect.

The needle antenna structures 30 shown in Fig. 1 may be inserted into other skin tissue structures that exist within the epidermis, the dermis and the subcutaneous tissue layers. To minimise patient discomfort and physical damage caused by antenna insertion, the maximum depth of physical penetration of the needle is between 0.1 mm and 10 mm, or more preferably 0.5mm and 2mm.

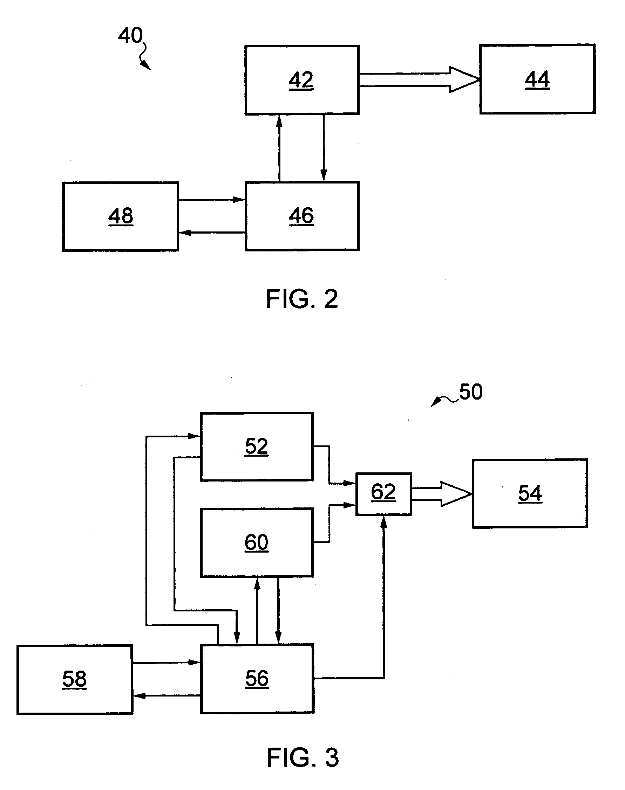

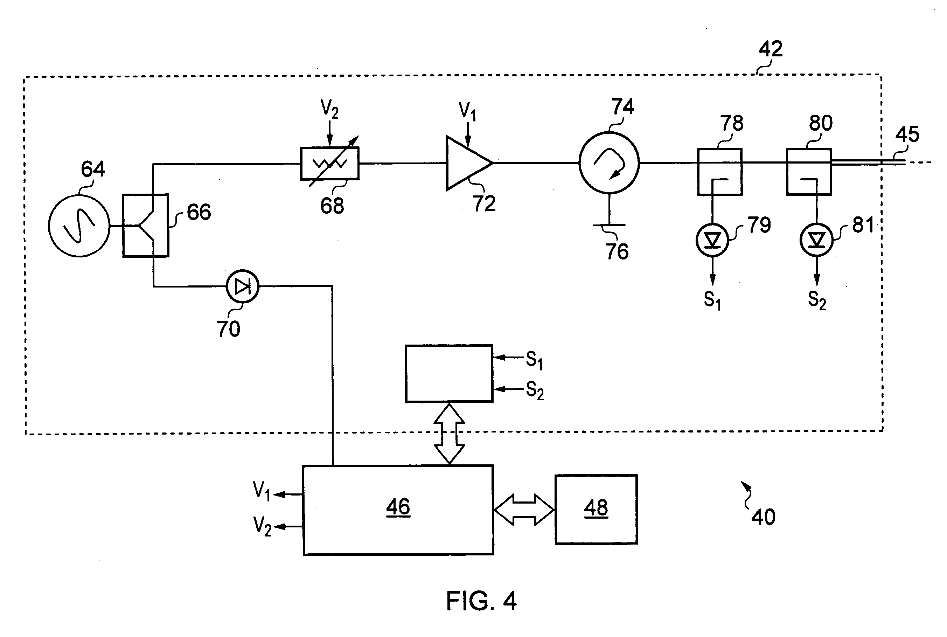

Fig. 2 is a block diagram showing skin treatment apparatus 40 that is an embodiment of the invention. The apparatus 40 comprises a signal generator 42 connected to a monopole antenna 44 such as the needle antenna discussed above. The signal generator 42 is also connected to a microprocessor or digital signal processor (DSP) 46 which is arranged to control the signal generator 42. A user interface 48 is connected to the DSP 46 to receive and display information about the treatment and to permit instructions e.g. control instructions from a user to be communicated to the DSP 46. The signal generator 42 is arranged to generate an electromagnetic signal with a frequency of 10 GHz or more and a power level such that the energy delivered into skin tissue at the radiating portion of the monopole antenna 44 is 10 mW or more. Details of components within the signal generator are discussed below with reference to Fig. 4.

Fig. 3 is a block diagram showing skin treatment and measurement apparatus 50 that is another embodiment of the invention. The apparatus 50 has two operation modes: a treatment mode, in which it operates in the same way as apparatus 40 discussed with reference to Fig. 2, and a measurement mode, in which a reflected signal from the antenna is used to measure dielectric properties or complex impedance of tissue at the radiating portion of the antenna e.g. to identify the tissue type into which the needle has been introduced. Referring back to Fig. 1, the measurement mode may be used to ensure that the needle antennas 30 are properly introduced into the sweat gland 28 or the sebaceous gland 26 and not into any adjacent structures. If the measured dielectric properties or complex impedance indicates that the needle is in the correct tissue type, treatment may begin, i.e. a higher level of power may be delivered to the antennas.

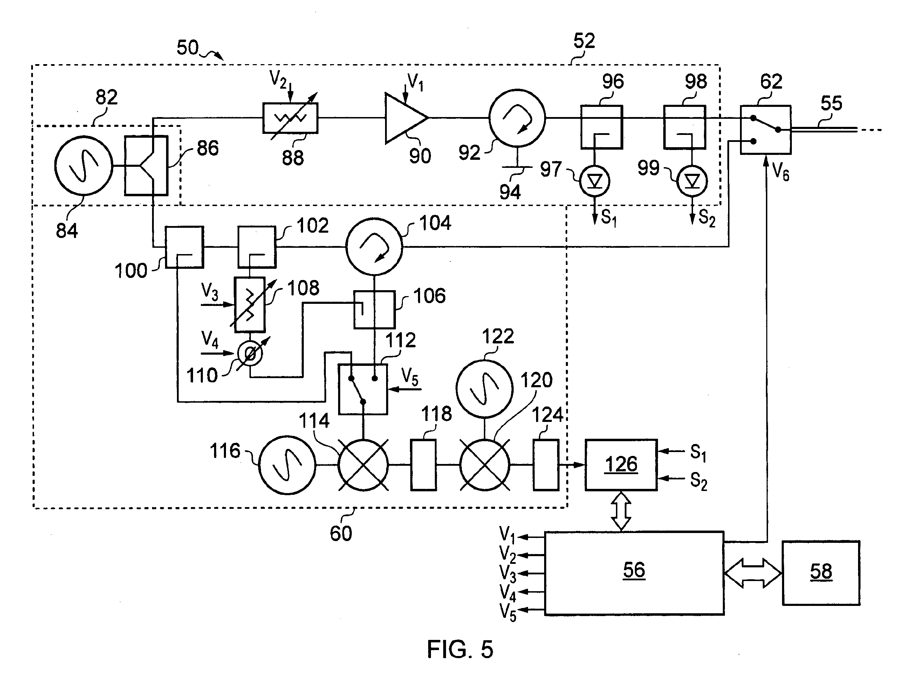

Returning to Fig. 3, the apparatus 50 include a signal generator 52, monopole antenna 54, DSP unit 56 and user interface 58 which correspond to the components having the same name in the discussion of Fig. 2 above. In addition, there is a measurement signal generator 60 for generating a signal with a low power level (e.g. less than 1 mW). In one embodiment, both signal generators may share the same source oscillator. In other embodiments different sources may be used e.g. so that different frequencies are used for treatment and measurement. For example, the apparatus may use a treatment frequency of 200 GHz and a measurement frequency of 500 GHz. The measurement signal generator may provide a different channel to the antenna, which channel bypasses the high power generation components of the signal generator 52. A switch 62, e.g. a low loss waveguide switch, controllable by the DSP unit 56 is connected between the antenna 54 and the signal generators 52, 60 to select which signal is sent to the antenna 54. The apparatus 50 thus operates in either the measurement mode or the treatment mode. Details of components within the signal generators are discussed below with reference to Fig. 5.

Fig. 4 is a block diagram showing the apparatus 40 of Fig. 2 with the components of the signal generator 42 shown in more detail. Components in common between Figs. 2 and 4 are given the same reference number.

The signal generator 42 comprises a source oscillator 64, which produces low level energy at a frequency within the range deemed to be of interest for implementing the current invention, i.e. more than 10 GHz, preferably between 30 GHz and 5 THz. The output from source oscillator 64 is connected to a power splitter 66, which splits the source power into two parts, which may be balanced (or equal amplitude) or may be unbalanced, i.e. 1/3 and 2/3. A first part is fed into a detector 70, e.g. a diode detector, whose output is fed to the DSP unit 46 to monitor the status of the source oscillator 64 to ensure that it is functioning correctly. The detector 70 may use a Schottky diode, i.e. a zero bias Schottky diode, or a tunnel diode. A second part is fed into a variable attenuator 68, which may be a PIN attenuator, whose attenuation is controlled by signal $V_2$ output from the DSP unit 46.

The output from the variable attenuator 68 is fed into the input port of the power amplifier 72 which amplifies or boosts the signal produced by the source oscillator 64 to a level that is useful for treating the biological (i.e. skin) tissue structures that are of interest. The power amplifier 72 is controllable by signal $V_1$ output from the DSP unit 46. A first port of a mm-wave circulator 74 is connected to the output stage of the power amplifier 72 to protect the amplifier from high levels of reflected power which may result from an impedance mismatch between the biological tissue and the radiating section of the antenna. A second port of the circulator 74 is connected to permit the forward (amplified) signal to travel to the antenna. Any reflected signals from the antenna therefore arrive at the second port, which is then diverted or directed to the third port. The third port of the circulator 74 is connected to a power dump load 76. The impedance of the power dump load 76 is selected such that all, or a high percentage, of the power reflected back into the second port of the circulator 74 is diverted to the third port, where its energy is dumped into the load. In one embodiment the impedance of the dump load is 50 $\Omega$, but it is not limited to this value. Preferably the impedance is equal to the characteristic impedance of the microwave components used in the system.

The second port of the circulator 74 is connected to a first directional coupler 78, which is configured as a forward power coupler and is used to sample a portion of the forward going power to enable the power level to be monitored. A coupling factor of between -10 dB and -30 dB may be used, which allows between 10% and 0.1% respectively of the main line power to be sampled. To preserve as much of the main line power as possible the coupling factor is preferably between -20 dB and -30 dB. The output from the coupled port of the first directional coupler 78 is connected to a detector 79 (e.g. diode detector) which converts that output to a DC or lower frequency AC signal $S_1$ and feeds it to the DSP unit 46. The detected forward power level may be processed by the DSP and displayed on the user interface 48. The location of first directional coupler 78 is not limited to the second port of circulator 74, i.e. it may be connected to the first port of circulator 74.

The main line output from the first directional coupler 78 is fed into the input port of a second directional coupler 80, which is configured as a reflected (or reverse) power coupler and is used to sample a portion of the reflected power to enable the level of returned or reflected power to be monitored and provide an indication of the impedance match (or mismatch) between the biological tissue and the radiating portion (distal tip or aerial) of the needle antenna. The output from the coupled port of the second directional coupler 80 is connected to a detector 81 (e.g. diode detector, homodyne detector or heterodyne detector) which converts that output to a DC or lower frequency AC signal $S_2$ , which may contain magnitude or magnitude and phase information, and feeds it to the DSP unit 46. The detected reflected power level may be processed by the DSP and displayed on the user interface 48.

The DSP unit 46 may be arranged to calculate and display, using the user interface 48, the net power being delivered into the tissue, e.g. by subtracting the reflected power level from the forward power level, taking into account the loss (insertion loss) of a delivery cable or PCB track 45 (e.g. a flexible co-axial cable, a flexible/twistable waveguide, a microstrip line, or a coplanar line) connected between the output port of the second directional coupler 80 and the input to the needle antenna, and the insertion loss of the needle antenna itself, i.e.

$$P_{net} = P_{forward} - P_{ch\_loss} - P_{ant\_loss} - P_{reflected}$$

where $P_{net}$ is net power, $P_{forward}$ is forward power, $P_{ch\_loss}$ is delivery channel loss, $P_{ant\_loss}$ is antenna structure loss, and $P_{reflected}$ loss due to reflected power caused by an impedance mismatch between the radiating section of the antenna and the biological tissue load.

The DSP unit 46, which may alternatively be a microprocessor, microcontroller, combined microprocessor and DSP unit, a single board computer or a single board computer and a DSP unit, may be used to control the functionality and operation of the apparatus. The DSP unit 46 may be responsible for controlling the variable attenuator 48, checking the status of the source oscillator 64, measuring the forward and reflected power levels, calculating the net power, generating user information and flagging up error conditions. The user interface 48 may include an input/output device arranged to enable the user to enter information into the system and for displaying parameters that may be of interest to the user. The input/output device may be a touch screen display unit, a keyboard/keypad and a LED/LCD display, LED segments and switches, or any other suitable arrangement for an input/output device.

The apparatus may include a DC isolation barrier (not shown here) connected between the generator and the patient to prevent a DC voltage path between the generator and the patient. Such a barrier may take the form of a microstrip capacitor or two sections of waveguide sandwiched between a sheet of low loss dielectric material, for example, a thin layer of microwave ceramic, Kapton® sheet or PTFE.

Fig. 5 is a block diagram showing the apparatus 50 of Fig. 3 with the components of the signal generator 52 and measurement signal generator 60 shown in more detail. Components in common between Figs. 3 and 5 are given the same reference number. Thus, selection of a treatment mode or measurement mode is made using signal $V_6$ from DSP unit 56 to switch 62, which causes a common switch contact to toggle between a contact connected to a treatment signal generator 52 (i.e. a microwave component line-up or sub-assembly for generating a treatment signal) and a contact connected to the measurement signal generator 60 (or microwave component line-up or sub-assembly) to select which signal is transmitted to the antenna along cable 55. The switch 62 is a single pole-two throw arrangement, and preferably introduces a minimal amount of attenuation of the signal passing through it, i.e. the loss through the switch may be less than 0.2 dB. The switch 62 may be a waveguide switch or a co-axial switch. For the upper frequency range disclosed herein a waveguide switch is preferred because it has a lower insertion loss. The waveguide switch basically enables two pieces of waveguide to be moved to enable the energy from either the measurement or treatment circuits to be connected to a common channel comprising of a cable assembly (or microstrip/coplanar line) and the antenna.

In Fig. 5, a common frequency source oscillator 82 is used by both the treatment signal generator 52 and the measurement signal generator 60. The frequency source 82 comprises a source oscillator 84 whose output is connected to a power splitter 86 (e.g. 3 dB power splitter or power coupler), which routes a first part of the signal to the treatment signal component line-up for operation in the treatment mode. A second part is routed to the measurement signal component line-up for operation in the measurement mode.

The treatment signal component line-up is similar to the signal generator 42 discussed above with reference to Fig. 4. Components with the same name perform a corresponding function. Thus, the treatment signal generator 52 includes a variable attenuator 88 connected to receive a signal from power splitter 86, a power amplifier 90, a circulator 92 arranged to isolate the power amplifier 90 from reflected signals, a power dump load 94 for receiving energy from reflected signals in the treatment mode, a forward directional coupler 96 which couples forward power to a detector 97, and a reverse directional coupler 98 which couples reflected power to a detector 99.

In a further embodiment, a tuning or matching circuit (not shown) may be connected between the output of the power amplifier 90 and the switch 62 in order to dynamically impedance match the tissue impedance seen by the antenna with the impedance of the signal generator 52 to provide maximum power transfer into the tissue. This arrangement will increase the efficiency between the microwave power delivered and the microwave power available from the source. This may be extremely advantageous where very high microwave or mm-wave or sub-mm wave frequency energy is used for treatment, since it is extremely expensive to generate high levels of energy at these frequencies, thus it is undesirable to lose even a small portion of this energy. This feature is also desirable when the delivered energy levels are required to cause relatively large volume ablation of tissue. For the smaller scale treatment considered herein, this feature may be optional. However, if this feature is implemented, the tuning circuit preferably uses varactor or PIN diodes as tuning elements rather than mechanical tuning rods or screws; this is due to physical size constraints. The detectors 97, 99 may be configured as a heterodyne detector to measure phase and magnitude information to control the tuning elements.

The measurement signal component line-up is provided on a separate signal line (e.g. channel) from the treatment signal component line-up. This bypasses the power amplifier 90 and other potentially noisy components which may affect measurement sensitivity. It also means that the measurement signal does not enter the detector via the coupled port of a directional coupler, and so is not limited by the returned signal being attenuated by the coupling factor of the coupler before reaching the input to the detector. Thus, the measurement signal generator 60 includes a reference directional coupler 100 connected to receive a signal from power splitter 86 into its input port. The reference directional coupler 100 is used to couple a portion of the forward power to provide a reference for the tissue measurement system. Depending upon the power level available from the power splitter 86, it may be necessary to include a low noise low power amplifier to boost the amplitude of the measurement signal. If this is required then the boost amplifier may be inserted between the power splitter 86 and the reference directional coupler 100.

The coupled signal from of the reference directional coupler 100 is connected to a first terminal of an electronically controlled single pole-two throw switch 112 (controlled by signal $V_5$ from DSP unit 56), whose function is to either route that coupled signal (hereinafter referred to as the "reference signal") or a reflected signal to the input of a heterodyne receiver where magnitude and phase information relating to the reference signal and the reflected signal is extracted.

The main output from the reference directional coupler 100 is input to a carrier directional coupler 102 which samples a further portion of the forward transmitted power signal for use in a circuit that provides carrier cancellation or increased isolation between the forward transmitted and the reflected measurement signals. In this embodiment, the carrier cancellation circuit provides enhanced isolation between the first and third ports of a low power circulator 104 which isolates the reflected signal from the forward signal.

The main output from the carrier directional coupler 102 is connected to the first port of the circulator 104. The second port of the circulator 104 is connected to the switch 62 to cause the forward directed signal from the source to be transferred along the cable 55 and along the antenna into the tissue. The second port of the circulator also receives a reflected signal from the antenna (via cable 55 and switch 62). The circulator 104 is arranged to divert the reflected signal to its third port, thereby isolating it from the forward signal received at the first port.

The reflected signal coming out of the third port of the circulator enters the input port of an isolation directional coupler 106, which injects into the main line a signal that is in anti-phase with any forward signal that breaks though the isolation between the first and third ports of the circulator 104 to enter the third port. The injected signal is known as the carrier cancellation signal and is generated from a coupled carrier signal from the carrier directional coupler 102. The coupled carrier signal is input to a variable attenuator 108 (controlled in this embodiment by signal $V_3$ from DSP unit 56; in other embodiments a manually adjustable attenuator may be used) which adjusts the amplitude of the carrier signal so that the injected signal has an amplitude equal to the unwanted signal coming out of the third port of the circulator towards the input to the detector. The output from the variable attenuator 108 is input to a variable phase adjuster 110 (controlled in this embodiment by signal $V_4$ from DSP unit 56; in other embodiments a manually adjustable phase shifter may be used) which adjusts the phase of the carrier signal to ensure that there is a 180° phase shift between the unwanted component of the signal from the third port of the circulator 104 and the injected signal. The phase adjuster 110 may be an electronically controlled device, for example, a PIN diode adjuster or a mechanically controlled adjuster, for example, a co-axial trombone.

The cancellation circuit may be set up by adjusting the phase and magnitude of the variable attenuator and phase adjuster with a representative cable assembly fitted; this will ensure that changes in phase and magnitude caused by the cable will also be cancelled out. By careful adjustment of the phase and magnitude of the signal injected into the coupled port of the third directional coupler, the unwanted signal component may be completely cancelled out and so the signal output from the isolation directional coupler 106 may be solely due to an impedance mismatch between the needle antenna and the tissue. This arrangement increases the measurement sensitivity of the overall measurement system.

The output of the isolation directional coupler 106 is provided to a second terminal of switch 112 where it is selectively received by a heterodyne receiver according to the selected switch configuration.

In this embodiment, the heterodyne receiver comprises a double IF heterodyne detector which is arranged to extract phase and magnitude information from the reference signal and the reflected signal. As mentioned above, the DSP unit 56 generates a signal $V_5$, which controls the configuration of the switch 112 to route either the reference signal or the reflected signal into the heterodyne receiver. The switch may be a PIN switch of either a reflective or an absorptive type, or a co-axial switch.

The output of switch 112 is connected to the RF input of a first frequency mixer 114. A first local oscillator 116 is connected to deliver a signal to the LO input of the first frequency mixer 114. The output signal from the first frequency mixer 114 (which is a first intermediate frequency) therefore comprises a signal having a frequency corresponding to the difference between the frequencies of the first local oscillator signal and the input (reflected or reference) signal.

The output signal from the first frequency mixer 114 is fed into a first low pass filter 118, whose function is to ensure that only the difference frequency produced by the first frequency mixer 114 is allowed to pass to the next component in the chain, i.e. the signal that is the sum of the two input frequencies and any other unwanted signals are filtered out.

The output from the first low pass filter 118 is fed into the RF input of a second frequency mixer 120. A second local oscillator 122 is connected to the LO input of the second frequency mixer 120. The output signal from the second frequency mixer 120 (which is a second intermediate frequency) therefore comprises a signal having a frequency corresponding to the difference between the frequencies of the second local oscillator signal and the first intermediate signal.

The output signal from the second frequency mixer 120 is fed into a second low pass filter 124, whose function is to ensure that only the difference frequency produced by the second frequency mixer 120 is passed to the next component in the chain.

The output of the second low pass filter 124 is fed into an analogue to digital converter (ADC) 126, whose function is to convert the analogue signal produced by the heterodyne receiver into a digital format to enable it to be processed by the DSP unit 56. It may be necessary to use more than two stages to reduce the mm-wave or sub-mm wave frequency used to perform the tissue measurement to a frequency that can be used by a standard analogue to digital converter in order to be able to effectively extract the required phase and magnitude information from the signal. According, the down-conversion of the primary signal may occur in a plurality of stages, e.g. more than the two stages described above. For example, a down conversion system that uses six frequency mixers, six low pass filters and six local oscillators may be configured as follows:

- Reflected signal frequency = 200 GHz
- First RF input (RF1) = reflected signal = 200 GHz
- First local oscillator signal (LO1) = 40 GHz
- First filtered intermediate signal (IF1)
  = RF1 - LO1 = 160 GHz
- Second RF input (RF2) = IF1 = 160 GHz
- Second local oscillator signal (LO2) = 40 GHz
- Second filtered intermediate signal (IF2)
  = RF2 - LO2 = 120 GHz
- Third RF input (RF3) = IF2 = 120 GHz
- Third local oscillator signal (LO3) = 40 GHz
- Third filtered intermediate signal (IF3)
  = RF3 - LO3 = 80 GHz
- Fourth RF input (RF4) = IF3 = 80 GHz
- Fourth local oscillator signal (LO4) = 40 GHz
- Fourth filtered intermediate signal (IF4)
  = RF4 - LO4 = 40 GHz
- Fifth RF input (RF5) = IF4 = 40 GHz
- Fifth local oscillator signal (LO5) = 39 GHz
- Fifth filtered intermediate signal (IF5) = RF5 - LO5 = 1 GHz
- Sixth RF input (RF6) = IF5 = 1 GHz
- Sixth local oscillator signal (LO6) = 950 MHz
- Sixth filtered intermediate signal (IF6) = RF6 - LO6 = 50 MHz

The sixth filtered intermediate signal produced by the heterodyne detector is at a sufficiently low enough frequency to enable it to be used by a standard ADC unit. The first four local oscillator signals may be derived from the same frequency source combined with an appropriate power splitter.

The DSP unit 56 is used to digitally extract the phase and magnitude information from both the reference signal and the reflected power measurement signal and to calculate the complex impedance (or other desired properties) of the tissue that is in contact with the distal tip of the needle antenna.

The frequencies of the first and second local oscillators 116, 122 may be synchronised with the source oscillator 84 to minimise any adverse effects caused by relative frequency drift between the oscillators. Moreover, synchronising the local oscillators to the measurement frequency enables the phase changes in the system to be referenced to a single source.

A single port calibration may be performed at the distal end of the antenna. This may be achieved by connecting a plurality of loads to the end of the antenna and running a calibration routine. It may be preferable to immerse the antenna into a plurality of liquid loads, each with a different, but repeatable, characteristic impedance. It may also be desirable to use a plurality of solid loads or loads made from grinding a solid material into dust or a powder that will enable the radiating section of the antenna to be surrounded. A mathematical routine can then be run that enables a one port calibration to be performed with three loads that differ in impedance, but are repeatable in value. The calibration required for this system is somewhat similar to the calibration routine performed by a vector network analyser, where it is required to attach a well defined open circuit, a short circuit and a 50 $\Omega$ load to the end of a standard test cable. The calibration routine used here is more complex in that the needle antenna does not lend itself well to having three standard loads attached to it, hence a plurality of liquids or powders may provide a useful solution to this problem. Once three repeatable loads are found then it is possible to perform a single port calibration and map the measurements onto a Smith Chart. The Smith chart is used to conveniently show any value of complex impedance. Certain tissue types or tissue states are then recognised by specific complex impedance values shown on the chart.

The apparatus may be activated using footswitch or handpiece control (not shown) connected to the DSP unit 56.

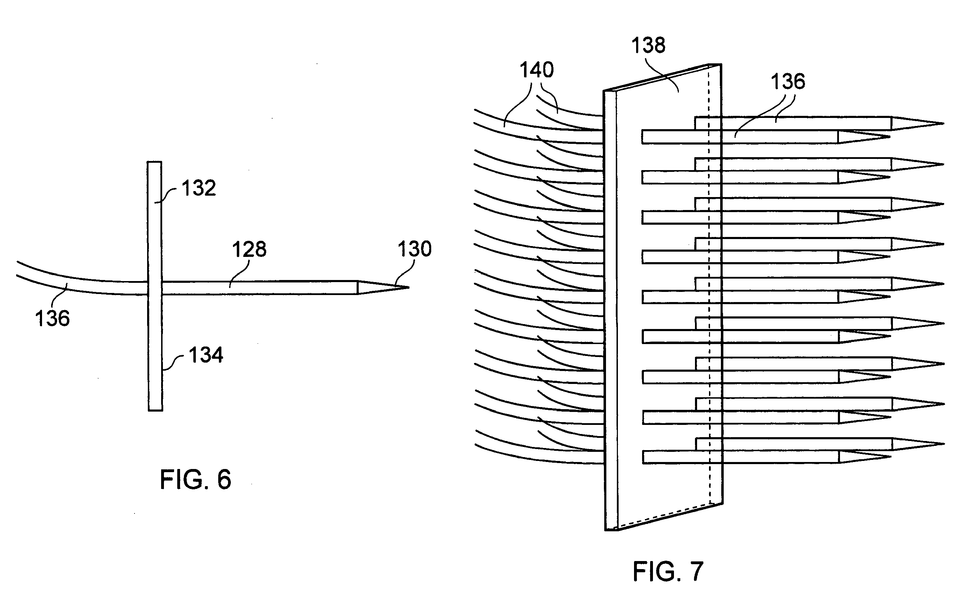

Fig. 6 shows an antenna structure that is suitable for use with an embodiment of the invention. The structure comprises a single monopole antenna 128 in the form of a needle, i.e. with a sharpened distal end 130. The needle may be formed on a rigid biocompatible material or may be made from stainless steel with a thin biocompatible coating, e.g. or Parylene C or the like. The antenna 128 is attached to and projects from a patch 132. The patch may be a sticky patch (i.e. with a layer of adhesive on its distal surface 134) for attaching the antenna structure to the surface of the skin. A cable assembly 136 carrying the electromagnetic signal (e.g. corresponding to cables 45 and 55 discussed above) may be attached to the proximal end of the needle through the patch. The needle is preferably less than 2 mm in length, and its diameter is preferably less than 0.5 mm.

Fig. 7 shows another antenna structure that is suitable for use with an embodiment of the invention. This structure comprises a regular array of needle antennas 136, each attached to and projecting from a sticky patch 138 in a similar way to the arrangement shown in Fig. 6. In this embodiment each antenna 136 has its own cable 140 attached to its proximal end. The sticky patch may be made from a flexible material to allow it to conform to the skin surface.

A large array of pin or needle antennas on a pad or flexible patch may be particular advantageous when used with apparatus capable of carrying out both measurement and treatment. In such an embodiment, each needle antenna may have its own independently controllable measurement and treatment signal generators. Measurements may be obtained for all antennas in the array and then only those antennas that are detected to be in the tissue structures of interest (e.g. sweat or sebaceous glands) may be switched to treatment mode or energised with enough microwave or mm-wave, or sub-mm wave energy to affect the tissue structure.

Additionally or alternatively, the apparatus may include a mechanism that moves individual antennas relative to the patch either to insert them deeper into the skin structure or to withdraw them completely. The movement may be controlled in accordance with the measured information. A piezoelectric or magnetostrictive material or a linear motor arrangement may be used to move the individual pins or a cluster of pins.

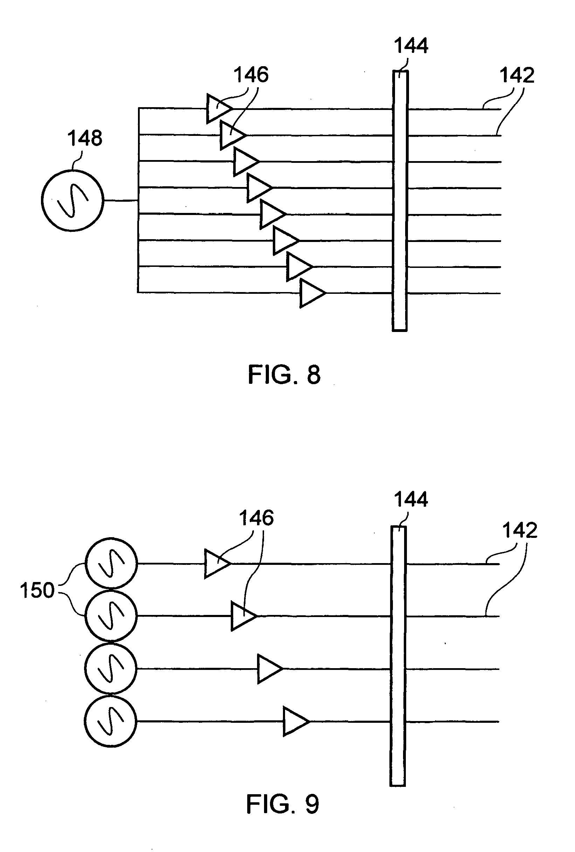

Fig. 8 shows a schematic view of an apparatus that is an embodiment of the invention in which each antenna 142 in an array of antennas attached to a flexible patch 144 has a dedicated signal line with an independently controllable amplifier 146 but where all the amplifiers have a common source oscillator 148. Fig. 9 illustrates an alternative configuration where each antenna has its own source oscillator 150.

In all embodiments discussed above, the power amplifiers may be mounted in close proximity to the needle or pin antennas. For example, they may be mounted in a layer on top of the flexible patch (substrate) used to support the needle antenna array. It may be necessary to use driver amplifiers between the source oscillator and the power amplifiers in order to boost the signal level produced by the source oscillator. A plurality of power amplifiers may be driven using a single driver amplifier, for example, one driver amplifier could be used to drive four power amplifiers, such that an array of 40 drivers could be configured to drive 160 power amplifiers.

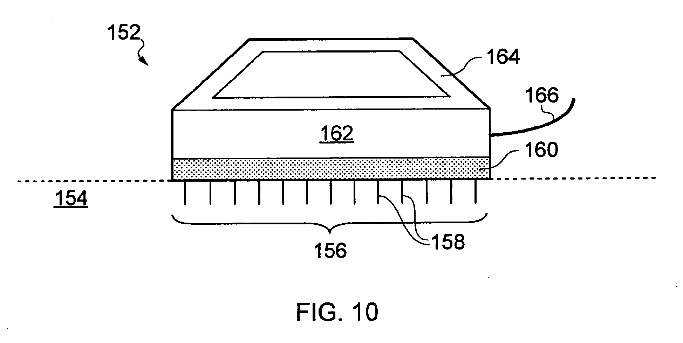

Fig. 10 is a schematic diagram of a physical arrangement for a complete instrument that may be used to implement an invasive or minimally invasive skin treatment system as described above. This arrangement may be particularly useful for the treatment of alopecia areata, where an array of needle antennae is introduced into the area of the scalp that requires treating.

The skin treatment instrument 152 comprises a self contained layered structure consisting of a sandwich of layers including: a needle antenna array 156 comprising a plurality of needle antennas 158 such as those discussed above, a substrate material 160, and a housing 162 containing further layers. The further layers may include an arrangement of mm-wave or sub-mm wave power transistors, and arrangement of driver transistors and power splitting networks, an arrangement of source oscillators, an arrangement of control circuits, a power supply system (this may be a battery pack and an arrangement of boost and/or buck converters or an external power cable 166), and a means of entering and displaying user information corresponding to the components of the apparatuses discussed above with respect to Figs. 4 and 5. The instrument may be gripped by a integral handle 164.

The treatment instrument 152 may be applied to the patient by placing it onto the surface of the scalp 154. The device may be held in place during treatment by using a handle arrangement that enables the surgeon to hold the device in position with ease whilst ensuring that patient discomfort is minimised.

The size of the array 156 may be developed to accommodate the amount of hair loss caused by alopecia in a particular patient, for example, the size may range from 1 cm$^2$ to 100 cm$^2$. The treatment of alopecia areata may also require a depth of penetration of mm-wave or sub-mm wave energy of between 0.2 mm and 2 mm. Thus, this embodiment may lend itself particularly well to this clinical application when frequencies in excess of 100 GHz, for example, 300 GHz or 500 GHz, are used.

This invention, especially the compact instrument shown in Fig. 10, is made possible through recent advances in microwave, millimetre and sub-millimetre wave power generation technology. Conventionally it has been impossible to generate power at the higher end of the microwave frequency band and beyond into the millimetre wave or sub-millimetre wave regions using semiconductor or solid state devices. Power generation at these frequencies was only previously possible using large tube based devices such as Klystrons, Magnetrons or devices based on a technique using Microwave Amplification by Stimulated Emission of Radiation (MASERs). These methods of power generation are highly impractical, for example, it can take a large room of equipment to generate up to 10 W of power at 200 GHz using a Klystron based system. In the implementation of such systems, water cooling and very large high voltage/current power supplies are required. These tube based sources also tend to be unstable and it can be difficult to control the level of power being delivered into tissue, i.e. the average power levels are normally controlled by changing the pulse width or the duty cycle of the power signals.

The invention draws in particular upon recent advances in microwave, millimetre and sub-millimetre wave monolithic integrated circuits (MMICs). For the successful implementation of new medical treatment devices associated with this invention, devices known as Indium Phosphide (InP) High Electron Mobility Transistors (HEMTs) are of particular interest. Recent developments in InP HEMT devices indicate that the technology is on the way to realising power devices that may be operated up to terahertz (1 THz = 1000GHz) frequencies. In the construction of InP HEMTs, indium phosphide is the substrate that the semiconductor InGaAs is grown onto. InGaAs shares the same lattice constant with InP. InP substrates tend to be small, for example 76 mm and have high dielectric constants, e.g. 12.4, which is close to that of GaAs.

GaAs pHEMT have emerged as a device of choice for implementing microwave and millimetre wave power amplifiers. In order to be able to achieve a high output power density, device structures with high current density and high sheet charge are required. The sheet charge density in a single heterojunction AlGaAs/InGaAs pHEMT is limited to $2.3 \times 10^{12}$ cm$^{-2}$, therefore a double heterojunction device structure must be used to increase the sheet charge

The millimetre wave power capability of single heterojunction AlInAs/GaInAs HEMTs has also been demonstrated. The requirements for suitable power HEMT devices are high gain, high current density, high breakdown voltage, low access resistance, and low knee voltage to increase output power and power added efficiency (PAE). The AlInAs/GaInAs/InP (InP HEMT) satisfies all of these requirements with the exception of high breakdown voltage. This limitation may be overcome by operating the device at a lower drain bias. The high gain and high PAE characteristics of InP HEMTs at low drain bias voltages make them ideal candidates for use in battery powered equipment. A further advantage of InP substrate is that it exhibits a 40% higher thermal conductivity than GaAs, thus allowing higher dissipated power per unit area of the device or lower operating temperature for the same power distribution. Therefore, it may be desirable to use InP HEMT devices to implement hand held treatment devices or to enable the devices to be mounted in close proximity to the monopole or pin antenna, thus a medical device that comprises a sandwich of layers may be fabricated. The high thermal conductivity may also allow a plurality of devices to be used to drive an array of radiating needle antennas. It may be possible to use a separate InP HEMT device to supply each pin or needle radiating structure.

Some specific examples of devices that may be used to implement the current invention are given below:

1. TRW Inc. have developed a production process based on 75 mm diameter InP substrates and 0.1 $\mu$m passivated InP HEMT devices that may operate up to 200 GHz;

2. Terabeam hxi Millimeter Wave Products (www.terabeam-hxi.com) manufacture a power module that produces up to 17 dBm (50 mW) of output power with a gain of 22 dB when operated at the 1 dB compression point over the frequency range of between 92 GHz and 96 GHz (model number: HHPAW-098);

3. Castle Microwave Limited currently represent a company that produces a W band power amplifier (part number:

AHP-94022624-01) to the following specification:

a. Centre operating frequency: 94 GHz
b. Bandwidth: +/- 1 GHz around 94 GHz
c. Typical saturated output power: 26 dBm (400 mW)
d. Minimum gain: 24 dB;

4. It has been shown that a n$^+$-p-n-n$^-$-n$^+$ wurtzite GaN structure may be operated at a frequency within the frequency range of between 230 GHz and 250 GHz to provide up to 350 mW of continuous wave power and up to 1.3 W of pulsed power (http://iop.org/EJ/02681242/16/9/311);

5. Northrop Grumman Space Technology (NGST) has developed a process for fabricating 0.1 $\mu$m InGaAs/InAlAs/InP HEMT MMICs on 100 mm InP substrates.

InP-based HEMT technology is a strong candidate for future high volume, high performance millimetre wave applications. The following reference provides details of InP-based HEMT devices that exhibit a cut-off frequency as high as 400 GHz: K. Shinohara, Y. Yamashita, A. Endoh, K. Hikosaka, T. Matsui, T. Mimura, and S. Hiyamizu, 'Ultrahigh-Speed Pseudomorphic InGaAs/InAlAs HEMTs With 400-GHz Cutoff Frequency', IEEE Electron Device Letters, Vol. 22, No. 11, pp. 507-509, Nov. 2001.

In summary, semiconductor device technologies that may be used to enable the current invention to be realised in practice include: mHEMT, pHEMT, MESFET, HBT, GaN. Full details of these and other similar device technologies may be found in the following text book: 'RF and Microwave Semiconductor Device Handbook', M. Golio, CRC Press, ISBN: 0-8493-1562-X. Chapters of particular interest: chapter 8 - High Electron Mobility Transistors, chapter 5 - Heterojunction Bipolar Transistors and chapter 7 - Metal Semiconductor Field Effect Transistors.

As mentioned above, a significant advantage of using high frequency millimetre wave energy or sub-millimetre wave energy for making tissue identification or state measurements is that the low power electromagnetic field produced by the antenna will be radiated over a very small distance that is local to the tip of the radiating needle antenna, hence the reflected or measurement signal will not be effected by adjacent layers of biological tissue. For example, if a measurement is to be made on dry skin and the layer of particular interest has an overall tissue thickness of 2 mm, and a frequency of 100 GHz is used to perform the measurement then the reflected signal obtained will be solely due to the skin tissue due to the fact that the penetration depth of microwave energy at 100 GHz in dry skin is 0.36 mm. On the other hand, if 20 GHz was to be used instead then the measured signal may suffer from interference or a signal component caused by adjacent tissue due to the fact that the depth of penetration at 20 GHz in dry skin is 1.38 mm. It should be noted that the depth of penetration is defined here as the distance travelled by the wave when its amplitude has been reduced to 37% of its initial launch amplitude.

Table 1 gives a list of the relevant electrical and dielectric properties associated with dry and wet skin at frequencies that may be of interest for implementing the current invention. These properties should be taken into account when designing suitable needle antenna structures.

Table 1: Tissue Parameters and monopole length requirements for dry and wet skin over a range of selected microwave frequencies

| Frequency (GHz) | Dry skin | | | | Wet skin | | | |
|---|---|---|---|---|---|---|---|---|
| | $\varepsilon_r$ | $\lambda$ (mm) | $\lambda/4$ (mm) | $d$ (mm) | $\varepsilon_r$ | $\lambda$ (mm) | $\lambda/4$ (mm) | $d$ (mm) |
| 20 | 21.96 | 3.20 | 0.8 | 1.38 | 23.77 | 3.08 | 0.77 | 1.39 |
| 30 | 15.51 | 2.57 | 0.64 | 0.85 | 17.74 | 2.37 | 0.59 | 0.88 |
| 40 | 11.69 | 2.19 | 0.55 | 0.65 | 14.09 | 2.00 | 0.5 | 0.67 |
| 45 | 10.40 | 2.07 | 0.52 | 0.59 | 12.81 | 1.86 | 0.47 | 0.605 |
| 50 | 9.40 | 1.96 | 0.49 | 0.54 | 11.77 | 1.75 | 0.44 | 0.56 |
| 60 | 7.98 | 1.77 | 0.44 | 0.48 | 10.22 | 1.56 | 0.39 | 0.49 |
| 70 | 7.04 | 1.62 | 0.41 | 0.43 | 9.12 | 1.42 | 0.36 | 0.43 |
| 80 | 6.40 | 1.48 | 0.37 | 0.40 | 8.32 | 1.30 | 0.33 | 0.40 |
| 90 | 5.94 | 1.37 | 0.34 | 0.38 | 7.72 | 1.20 | 0.30 | 0.37 |
| 100 | 5.60 | 1.27 | 0.32 | 0.36 | 7.25 | 1.11 | 0.28 | 0.35 |

The symbols given in the table above: $\varepsilon_r$, $\lambda$, $\lambda/4$ and $d$ represent relative permittivity, the loaded wavelength, quarter loaded wavelength (or the monopole antenna length), and depth of penetration respectively.

**Claims**

1. Skin treatment apparatus, comprising:

   a signal generator (42) arranged to output an electromagnetic signal having a frequency of 10 GHz or more and a reference signal;
   a monopole antenna (44) connected to receive the output electromagnetic signal, the monopole antenna having a penetrative structure (30) that is insertable into skin tissue (12, 14, 16), the penetrative structure including a radiating portion arranged to emit into the skin tissue a localised field of radiation corresponding to the received electromagnetic signal; and
   a measurement detector (81, 97, 99) connected between the signal generator (42) and the monopole antenna (44) to receive the reference signal and a reflected signal returning from the monopole antenna;
   wherein the measurement detector (81, 97, 99) is arranged to output a signal indicative of the magnitude and phase of the reflected signal relative to the reference signal, and
wherein the signal generator (42) is arranged to output the electromagnetic signal at a power level which permits the field of radiation to deliver energy into skin tissue at a power level of 10 mW or more, **characterised in that** the apparatus includes:

an antenna movement mechanism for moving the monopole antenna between a treatment position and a retracted position;
and
a processing device (46) in communication with the measurement detector (81, 97, 99) and the antenna movement mechanism, the processing device being arranged to:

calculate a complex impedance value for the tissue at the radiating portion of the monopole antenna based on the signal indicative of the magnitude and phase of the reflected signal relative to the reference signal output by the measurement detector, which complex impedance value is indicative of the tissue type; and
generate a control signal for the antenna movement mechanism based on the calculated complex impedance value,

wherein the antenna movement mechanism is arranged to move the monopole antenna between the treatment and retracted position based on the control signal.

2. Skin treatment apparatus according to claim 1,
wherein the penetrative structure (30) comprises a needle having a length that corresponds to an odd multiple of a quarter of the wavelength of the electromagnetic signal when the needle is in contact with a predetermined load impedance, the needle having a sharpened tip, and the radiating portion being located at the tip.

3. Skin treatment apparatus according to claim 2,
wherein the radiating portion extends for 1 mm or less along
the tip of the needle (30).

4. Skin treatment apparatus according to any one of the preceding claims, wherein the signal generator (42) includes a power level controller for adjustably controlling the power level of the output electromagnetic signal.

5. Skin treatment apparatus according to claim 4, including a delivered power detector (97, 99) connected between the signal generator (42) and the antenna (44), the delivered power detector being arranged to detect:

(i) a transferred power level indicative of the power transferred to the antenna from the signal detector, and
(ii) a reflected power level indicative of the power reflected back through the antenna from the radiating portion,
wherein the power level controller is arranged to adjustably control the power level of the output electromagnetic signal based on the transferred and reflected power levels detected by the delivered power detector.

6. Skin treatment apparatus according to claim 5, the processing device (46) being in communication with the delivered power detector (97, 99) and the power level controller, the processing device being arranged to:

receive signals indicative of the transferred and reflected power levels from the delivered power detector;
determine a net delivered power level indicative of the energy delivered into skin tissue at the radiating portion;

and
generate a control signal for the power level controller based on the determined net delivered power level,
wherein the power level controller is arranged to adjustably control the power level of the output electromagnetic signal based on the control signal from the processing device.

7. Skin treatment apparatus according to claim 6, comprising a plurality of monopole antennas (158), each monopole antenna being arranged to receive an output electromagnetic signal from the signal generator (42), and each monopole antenna (158) having a penetrative structure (30) that is insertable into skin tissue, the penetrative structure (30) including a radiating portion arranged to emit into the skin tissue a localised field of radiation corresponding to the received electromagnetic signal,
wherein the apparatus comprises a plurality of power level controllers and delivered power detectors, each monopole antenna having a respective power level controller and a delivered power detector associated therewith, each respective power level controller being independently controllable.

8. Skin treatment apparatus according to claim 7,comprising a handheld applicator structure (152), wherein the plurality of antennas (158) are mounted on and protrude from the handheld applicator structure, the applicator structure including the plurality of the power level controllers and the delivered power detectors, and wherein the signal generator (42) comprises a plurality of independently controllable power amplifiers (146) in the applicator structure, each antenna (158) being connected to receive the output electromagnetic signal from a respective power amplifier.

9. Skin treatment apparatus according to any preceding claim, wherein the output electromagnetic signal received by the monopole antenna has a power level of 10 mW or more.

10. Skin treatment apparatus according to claim 9, the processing device (46) being in communication with the measurement detector (81, 97, 99) and the signal generator (42), the processing device being arranged to:

calculate a complex impedance value for the tissue at the radiating portion of the antenna or of each antenna (158) based on the signal indicative of the magnitude and phase of the reflected signal relative to the reference signal output by the measurement detector, which complex impedance value is indicative of the tissue type; and generate a control signal for the signal generator (42) based on the calculated complex impedance value.

11. Skin treatment apparatus according to claim 10,
wherein the signal generator (42) is arranged to deliver an electromagnetic signal to the monopole antenna (44) or to each monopole antenna via either a treatment channel, which includes a power amplifier for increasing a power level of the output electromagnetic signal to 10 mW or more, or a measurement channel, which bypasses the power amplifier, wherein the electromagnetic signal from the signal generator (42) is switchable between the measurement channel to the treatment channel based on the generated control signal from the processing device (46).

12. Skin treatment apparatus according to claim 11,
wherein the electromagnetic signal delivered from the measurement channel has a power level two or more orders of magnitude less than the electromagnetic signal delivered from the treatment channel.

**Patentansprüche**

1. Hautbehandlungsvorrichtung, die Folgendes aufweist:

einen Signalgenerator (42) der eingerichtet ist,
ein elektromagnetisches Signal mit einer Frequenz von 10 GHz oder mehr und ein Referenzsignal auszugeben;
eine Monopolantenne (44), die zum Empfangen des elektromagnetischen Ausgangssignals geschaltet ist, wobei die Monopolantenne eine eindringende Struktur (30) aufweist, die in Hautgewebe (12, 14, 16) einführbar ist, wobei die eindringende Struktur einen Strahlungsabschnitt aufweist, der eingerichtet ist, ein örtlich begrenztes Strahlungsfeld, welches dem empfangenen elektromagnetischen Signal entspricht, in das Hautgewebe abzugeben; und
einen Messaufnehmer (81, 97, 99), der zwischen den Signalgenerator (42) und die Monopolantenne (44) geschaltet ist, um das Referenzsignal und ein reflektiertes Signal, das von der Monopolantenne zurückkommt, zu empfangen;
wobei der Messaufnehmer (81, 97, 99) eingerichtet ist, ein Signal auszugeben, das die Größe und Phase des reflektierten Signals im Verhältnis zu dem Referenzsignal angibt, und
wobei der Signalgenerator (42) eingerichtet ist,
das elektromagnetische Signal auf einem Leistungspegel auszugeben, welcher es dem Strahlungsfeld gestattet, Energie in Hautgewebe auf einem Leistungspegel von 10 mW oder mehr abzugeben,
**dadurch gekennzeichnet, dass** die Vorrichtung Folgendes umfasst:

einen Antennen-Bewegungsmechanismus zum Bewegen der Monopolantenne zwischen einer Behandlungsposition und einer eingezogenen Position; und
eine Verarbeitungsvorrichtung (46), die mit dem Messaufnehmer (81, 97, 99) und dem Antennen-Bewegungsmechanismus in Verbindung steht, wobei die Verarbeitungsvorrichtung eingerichtet ist:

zum Berechnen eines komplexen Widerstandswerts für das Gewebe an dem Strahlungsabschnitt der Monopolantenne auf der Grundlage des Signals, welches die Größe und Phase des reflektierten Signals im Verhältnis zu dem durch den Messaufnehmer ausgegebenen Referenzsignal angibt, wobei der komplexe Widerstandswert den Gewebetyp angibt; und

zum Erzeugen eines Steuersignals für den Antennen-Bewegungsmechanismus auf der Grundlage des berechneten komplexen Widerstandswerts,

wobei der Antennen-Bewegungsmechanismus eingerichtet ist, die Monopolantenne zwischen der Behandlungs- und der eingezogenen Position auf der Grundlage des Steuersignals zu bewegen.

2. Hautbehandlungsvorrichtung nach Anspruch 1, wobei die eindringende Struktur (30) eine Nadel mit einer Länge aufweist, die einem ungeradzahligen Vielfachen von einem Viertel der Wellenlänge des elektromagnetischen Signals entspricht, wenn sich die Nadel mit einem vorgegebenen Lastwiderstand in Kontakt befindet, wobei die Nadel eine angeschliffene Spitze aufweist und der Strahlungsabschnitt an der Spitze positioniert ist.

3. Hautbehandlungsvorrichtung nach Anspruch 2, wobei der Strahlungsabschnitt sich für 1 mm oder weniger entlang der Spitze der Nadel (30) erstreckt.

4. Hautbehandlungsvorrichtung nach einem der vorangehenden Ansprüche, wobei der Signalgenerator (42) einen Leistungspegelregler zum einstellbaren Steuern des Leistungspegels des elektromagnetischen Ausgangssignals aufweist.

5. Hautbehandlungsvorrichtung nach Anspruch 4, die einen Leistungsabgabe-Detektor (97, 99) umfasst, der zwischen den Signalgenerator (42) und die Antenne (44) geschaltet ist, wobei der Leistungsabgabe-Detektor eingerichtet ist zum Ermitteln von:

(i) einem Pegel der übertragenen Leistung, welcher die von dem Signaldetektor auf die Antenne übertragene Leistung angibt, und
(ii) einem Pegel der reflektierten Leistung, welcher die Leistung angibt, die von dem Strahlungsabschnitt durch die Antenne zurückreflektiert wird,
wobei der Leistungspegelregler eingerichtet ist, den Leistungspegel des elektromagnetischen Ausgangssignals auf der Grundlage der übertragenen und reflektierten Leistungspegel, die von dem Leistungspegel-Detektor detektiert werden, einstellbar zu steuern.

6. Hautbehandlungsvorrichtung nach Anspruch 5, wobei die Verarbeitungsvorrichtung (46) mit dem Leistungsabgabe-Detektor (97, 99) und dem Leistungspegelregler in Verbindung steht, wobei die Verarbeitungsvorrichtung eingerichtet ist:

zum Empfangen von Signalen, welche die übertragenen und reflektierten Leistungspegel des Leistungsabgabe-Detektors angeben;
zum Bestimmen eines Nettobetrages des Pegels der abgegebenen Leistung, welcher die Energie angibt, die an dem Strahlungsabschnitt an Hautgewebe abgegeben wurde; und
zum Erzeugen eines Steuersignals für den Leistungspegelregler auf der Grundlage des bestimmten Nettobetrages des Pegels der abgegebenen Leistung,
wobei der Leistungspegelregler eingerichtet ist,
den Leistungspegel des elektromagnetischen Ausgangssignals auf der Grundlage des Steuersignals der Verarbeitungsvorrichtung einstellbar zu steuern.

7. Hautbehandlungsvorrichtung nach Anspruch 6, die mehrere Monopolantennen (158) aufweist, wobei jede Monopolantenne eingerichtet ist, ein elektromagnetisches Ausgangssignal von dem Signalgenerator (42) zu empfangen, und jede Monopolantenne (158) eine eindringende Struktur (30) aufweist, welche in Hautgewebe einführbar ist, wobei die eindringende Struktur (30) einen Strahlungsabschnitt aufweist, der eingerichtet ist, ein örtlich begrenztes Strahlungsfeld, welches dem empfangenen elektromagnetischen Signal entspricht, in das Hautgewebe abzugeben, wobei die Vorrichtung mehrere Leistungspegelregler und Leistungsabgabe-Detektoren aufweist, jede Monopolantenne einen entsprechenden mit ihr verbundenen Leistungspegelregler und Leistungsabgabe-Detektor aufweist, wobei jeder entsprechende Leistungspegelregler unabhängig steuerbar ist.

8. Hautbehandlungsvorrichtung nach Anspruch 7, die eine handgehaltene Applikatoreinrichtung (152) aufweist, wobei die mehreren Antennen (158) auf der handgehaltenen Applikatoreinrichtung befestigt sind und aus ihr herausragen, die Applikatoreinrichtung mehrere Leistungspegelregler und Leistungsabgabe-Detektoren aufweist und wobei der Signalgenerator (42) mehrere unabhängig steuerbare Leistungsverstärker (146) in der Applikatoreinrichtung aufweist, wobei jede Antenne (158) geschaltet ist, das elektromagnetische Ausgangssignal von einem entsprechenden Leistungsverstärker zu empfangen.

9. Hautbehandlungsvorrichtung nach einem der vorangehenden Ansprüche, wobei das elektromagnetische Ausgangssignal, welches durch die Monopolantenne empfangen wird, einen Leistungspegel von 10 mW oder mehr aufweist.

10. Hautbehandlungsvorrichtung nach Anspruch 9, wobei die Verarbeitungsvorrichtung (46) mit dem Messaufnehmer (81, 97, 99) und dem Signalgenerator (42) in Verbindung steht, wobei die Verarbeitungsvorrichtung eingerichtet ist:

   zum Berechnen eines komplexen Widerstandswerts für das Gewebe an dem Strahlungsabschnitt der Antenne oder von jeder Antenne (158) auf der Grundlage des Signals, welches die Größe und Phase des reflektierten Signals im Verhältnis zu dem durch den Messaufnehmer ausgegebenen Referenzsignal angibt, wobei der komplexe Widerstandswert den Gewebetyp angibt; und
   zum Erzeugen eines Steuersignals für den Signalgenerator (42) auf der Grundlage des berechneten komplexen Widerstandswerts.

11. Hautbehandlungsvorrichtung nach Anspruch 10, wobei der Signalgenerator (42) eingerichtet ist, ein elektromagnetisches Signal an die Monopolantenne (44) oder an jede Monopolantenne entweder über einen Behandlungskanal, der einen Leistungsverstärker zur Erhöhung eines Leistungspegels des elektromagnetischen Ausgangssignals auf 10 mW oder mehr aufweist, oder einen Messkanal, welcher den Leistungsverstärker umgeht, abzugeben, wobei das elektromagnetische Signal von dem Signalgenerator (42) auf der Grundlage des erzeugten Steuersignals von der Verarbeitungsvorrichtung (46) zwischen dem Messkanal und dem Behandlungskanal umschaltbar ist.

12. Hautbehandlungsvorrichtung nach Anspruch 11, wobei das von dem Messkanal abgegebene elektromagnetische Signal einen Leistungspegel aufweist, der zwei oder mehr Größenordnungen niedriger ist, als das von dem Behandlungskanal abgegebene elektromagnetische Signal.

## Revendications

1. Appareil de traitement de la peau comprenant :

   - un générateur de signaux (42) conçu pour émettre un signal électromagnétique ayant une fréquence de 10 GHz ou plus et un signal de référence ;
   - une antenne monopôle (44) connectée de manière à recevoir le signal électromagnétique émis, laquelle antenne monopôle comprend une structure pénétrante (30) qui vient s'insérer dans les tissus de la peau (12, 14, 16), laquelle structure pénétrante comprend une partie rayonnante conçue pour émettre dans les tissus de la peau un champ localisé de rayonnement correspondant au signal électromagnétique reçu ; et
   - un détecteur de mesure (81, 97, 99) connecté entre le générateur de signaux (42) et l'antenne monopôle (44) afin de recevoir le signal de référence et un signal réfléchi renvoyé par l'antenne monopôle ;
   - dans lequel le détecteur de mesure (81, 97, 99) est conçu pour émettre un signal indiquant la magnitude et la phase du signal réfléchi par rapport au signal de référence ; et
   - dans lequel le générateur de signaux (42) est conçu de manière à émettre le signal électromagnétique à un niveau de puissance qui permet au champ de rayonnement de fournir une énergie dans les tissus de la peau à un niveau de puissance de 10 mW ou plus ;
   - **caractérisé en ce que** l'appareil comprend :
   - un mécanisme de déplacement d'antenne afin de déplacer l'antenne monopôle entre une position de traitement et une position rétractée ; et
   - un dispositif de traitement (46) en communication avec le détecteur de mesure (81, 97, 99) et le mécanisme de déplacement d'antenne, lequel dispositif de traitement est conçu pour :
   - calculer une valeur d'impédance complexe pour les tissus au niveau de la partie rayonnante de l'antenne monopôle en fonction du signal indiquant la magnitude et la phase du signal réfléchi par rapport au signal de référence émis par le détecteur de mesure, laquelle valeur d'impédance complexe indique le type de tissus ; et
   - générer un signal de commande pour le mécanisme de déplacement d'antenne en fonction de la valeur d'impédance complexe calculée ;

- dans lequel le mécanisme de déplacement d'antenne est conçu pour déplacer l'antenne monopôle entre la position de traitement et la position rétractée en fonction du signal de commande.

2. Appareil de traitement de la peau selon la revendication 1, dans lequel la structure pénétrante (30) comprend une aiguille ayant une longueur qui correspond à un multiple impair d'un quart de la longueur d'onde du signal électromagnétique lorsque l'aiguille est en contact avec une impédance de charge prédéterminée, laquelle aiguille possède une pointe pointue et la partie rayonnante se situe au niveau de la pointe.

3. Appareil de traitement de la peau selon la revendication 2, dans lequel la partie rayonnante s'étend sur 1 mm ou moins le long de la pointe de l'aiguille (30).

4. Appareil de traitement de la peau selon l'une quelconque des revendications précédentes, dans lequel le générateur de signaux (42) comprend une unité de commande de niveau de puissance pour commander et ajuster le niveau de puissance du signal électromagnétique émis.

5. Appareil de traitement de la peau selon la revendication 4, comprenant un détecteur de puissance fournie (97, 99) connecté entre le générateur de signaux (42) et l'antenne (44), lequel détecteur de puissance fournie est conçu pour détecter :

- (i) un niveau de puissance transférée indiquant la puissance transférée vers l'antenne depuis le détecteur de signaux ; et
- (ii) un niveau de puissance réfléchie indiquant la puissance réfléchie en retour à travers l'antenne depuis la partie rayonnante ;
- dans lequel l'unité de commande de niveau de puissance est disposée de manière à commander et ajuster le niveau de puissance du signal électromagnétique émis en fonction des niveaux de puissance transférée et réfléchie détectés par le détecteur de niveau de puissance.

6. Appareil de traitement de la peau selon la revendication 5, dans lequel le dispositif de traitement (46) est en communication avec le détecteur de puissance fournie (97, 99) et l'unité de commande de niveau de puissance, lequel dispositif de traitement est conçu pour :

- recevoir des signaux indiquant les niveaux de puissance transférée et réfléchie depuis le détecteur de puissance fournie ;
- déterminer un niveau de puissance fournie net indiquant l'énergie envoyée dans les tissus de la peau au niveau de la partie rayonnante ; et
- générer un signal de commande pour l'unité de commande de niveau de puissance en fonction du niveau de puissance fournie net déterminé ;
- dans lequel l'unité de commande de niveau de puissance est conçue pour commander et ajuster le niveau de puissance du signal électromagnétique émis en fonction du signal de commande du dispositif de traitement.

7. Appareil de traitement de la peau selon la revendication 6, comprenant plusieurs antennes monopôles (158), chaque antenne monopôle étant disposée de manière à recevoir un signal électromagnétique émis depuis le générateur de signaux (42) et chaque antenne monopôle (158) comprenant une structure pénétrante (30) venant s'insérer dans les tissus de la peau, la structure pénétrante (30) comprenant une partie rayonnante conçue pour émettre dans les tissus de la peau un champ localisé de rayonnement correspondant au signal électromagnétique reçu ;

- lequel appareil comprend plusieurs unités de commande de niveau de puissance et détecteurs de puissance fournie, chaque antenne monopôle ayant une unité de commande de niveau de puissance respective et un détecteur de puissance fournie qui lui est associé, et chaque unité de commande de niveau de puissance respective pouvant être commandée indépendamment.

8. Appareil de traitement de la peau selon la revendication 7, comprenant une structure d'applicateur tenue à la main (152), dans lequel lesdites plusieurs antennes (158) sont montées sur la structure d'applicateur tenue à la main de manière à en dépasser, laquelle structure d'applicateur comprend lesdites plusieurs unités de commande de niveau de puissance et les détecteurs de puissance fournie, et dans lequel le générateur de signaux (42) comprend plusieurs amplificateurs de puissance commandés indépendamment (146) dans la structure d'applicateur, chaque antenne (158) étant connectée de manière à recevoir le signal électromagnétique émis depuis un amplificateur de puissance respectif.

9. Appareil de traitement de la peau selon l'une quelconque des revendications précédentes, dans lequel le signal électromagnétique émis reçu par l'antenne monopôle possède un niveau de puissance de 10 mW ou plus.

10. Appareil de traitement de la peau selon la revendication 9, dans lequel le dispositif de traitement (46) est en communication avec le détecteur de mesure (81, 97, 99) et le générateur de signaux (42), lequel dispositif de traitement est conçu pour :

   - calculer une valeur d'impédance complexe pour les tissus au niveau de la partie rayonnante de l'antenne ou de chaque antenne (158) en fonction du signal indiquant la magnitude et la phase du signal réfléchi par rapport au signal de référence émis par le détecteur de mesure, laquelle valeur d'impédance complexe indique le type de tissus ; et
   - générer un signal de commande pour le générateur de signaux (42) en fonction de la valeur d'impédance complexe calculée.

11. Appareil de traitement de la peau selon la revendication 10, dans lequel le générateur de signaux (42) est conçu pour fournir un signal électromagnétique à l'antenne monopôle (44) ou à chaque antenne monopôle via un canal de traitement, qui comprend un amplificateur de puissance afin d'augmenter le niveau de puissance du signal électromagnétique émis à 10 mW ou plus, ou un canal de mesure qui contourne l'amplificateur de puissance, lequel signal électromagnétique provenant du générateur de signaux (42) peut être commuté du canal de mesure au canal de traitement en fonction du signal de commande généré venant du dispositif de traitement (46).

12. Appareil de traitement de la peau selon la revendication 11, dans lequel le signal électromagnétique fourni par le canal de mesure possède un niveau de puissance qui est inférieur de deux ordres de grandeur ou plus au signal électromagnétique fourni par le canal de traitement.

FIG. 1

FIG. 2

FIG. 3

FIG. 4

FIG. 5

FIG. 7

FIG. 6

144

146

148

142

**FIG. 8**

144

146

142

150

**FIG. 9**

152

164

166

162

160

154

156

158

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008071914 A
- WO 2004047659 A
- US 20080125775 A
- WO 2006078862 A
- WO 2008043997 A
- WO 2008043999 A
- WO 2008044000 A
- WO 2005115235 A
- WO 2008044013 A

**Non-patent literature cited in the description**

- **K. SHINOHARA ; Y. YAMASHITA ; A. ENDOH ; K. HIKOSAKA ; T. MATSUI ; T. MIMURA ; S. HIYAMI-ZU.** Ultrahigh-Speed Pseudomorphic InGaAs/InAlAs HEMTs With 400-GHz Cutoff Frequency. *IEEE Electron Device Letters,* November 2001, vol. 22 (11), 507-509
- RF and Microwave Semiconductor Device Handbook. **M. GOLIO.** High Electron Mobility Transistors, chapter 5 - Heterojunction Bipolar Transistors and chapter 7 - Metal Semiconductor Field Effect Transistors. CRC Press